# EUROPEAN PATENT APPLICATION

(11) **EP 4 643 793 A1**
(43) Date of publication of application: **05.11.2025**
(21) Application number: 23909916.1
(22) Date of filing: 30.11.2023
(51) Int. Cl.: A61B 17/12, A61M 25/10

(54) **BALLOON SHEATH CATHETER**

(30) Priority: 28.12.2022 CN 202211697414
(71) Applicant: Shenzhen Wecan Medical Technology Co., Ltd., Shenzhen, Guangdong 518063 (CN)
(72) Inventor: WU, Liping, Shenzhen, Guangdong 518000 (CN); HAO, Chenxiang, Shenzhen, Guangdong 518000 (CN); LI, Zhen, Shenzhen, Guangdong 518000 (CN); ZHANG, Xiong, Shenzhen, Guangdong 518000 (CN)
(74) Representative: Michalski Hüttermann & Partner Patentanwälte mbB
(86) International application number: PCT/CN2023/135416
(87) International publication number: WO 2024/139986

(57) **Abstract**

A balloon sheath (100), including a tubular main body (110) and a balloon (120) located at a distal end of the main body (110). The main body (110) internally comprises an inflation chamber (121). The inflation chamber (121) is communicated with the balloon (120), and an inner wall of the main body (110) can be driven to move towards an inner side when the balloon (120) expands. According to the balloon sheath (100), the inflation chamber (121) is arranged inside the main body (110), thereby reducing the outer diameter of the balloon sheath (100). In addition, when the inflation pressure of the balloon (120) gradually increases and is less than a preset value, the balloon (120) expands to occlude blood flow. At this point, the balloon (120) gradually expands to fill a gap between the main body (110) and a blood vessel, while still allowing an instrument to pass through the main body (110) normally. When the inflation pressure reaches the preset value, it means that the internal pressure of the balloon (120) reaches a strength capable of deforming a distal end section of the main body (110). At this point, the balloon (120) not only fills the gap between the main body (110) and the blood vessel, but also forces a corresponding position at the distal side of the main body (110) to squeeze and deform towards the inner side, such that the inner wall of the main body (110) is tightly attached to the instrument, thereby achieving the effect of simultaneously fixing the instrument and occluding the blood flow.

## Description

### Technical Field

The present invention relates to the technical field of interventional medical instruments, and particularly to a balloon sheath.

### Background Art

Since the 1970s, the vascular interventional therapy technology has been developed rapidly and vigorously. Sheaths play an important role in establishing a passage for surgical instruments. However, many vascular interventional surgeries require the occlusion of blood flow, which has led to the emergence of balloon sheaths.

As for the current balloon sheaths, their structures typically include a layer of outer membrane covering the sheath to construct an annular inflation chamber, so that the outer diameter of the product is too large, which is not conducive to protection of the inner wall of the blood vessel and wound healing. In addition, the current sheaths only serve to establish a passage for instruments and are unable to lock the instrument within the passage or provide hemostasis.

In conclusion, to optimize the existing balloon sheaths, it is very important to develop a balloon sheath with a smaller outer diameter and a locking function.

### Summary of the Invention

Based on this, it is necessary to provide an improved balloon sheath to address the issues of the existing balloon sheaths, such as their excessively large outer diameter and lack of a locking function.

A balloon sheath is provided, including a tubular main body and a balloon located at a distal end of the main body. The main body internally includes an inflation chamber. The inflation chamber is communicated with the balloon, and an inner wall of the main body can be driven to move towards an inner side when the balloon expands.

In an embodiment, the main body includes an inner layer and an outer layer, and a spring tube is arranged between the inner layer and the outer layer.

In an embodiment, the spring tube avoids an area of the main body where the balloon is located.

In an embodiment, the balloon is communicated with an outer surface of the inner layer.

In an embodiment, a plurality of balloons are arranged, and the plurality of balloons are distributed in a circumferential direction at the distal end of the main body.

In an embodiment, the main body includes at least one intermediate layer, and the intermediate layer at least partially covers an area where the balloon is located in a circumferential direction.

In an embodiment, the intermediate layer includes one or more of a spring tube, a spring sheet, or a mesh sheet.

In an embodiment, a plurality of intermediate layers are arranged, and the intermediate layers include a first intermediate layer and a second intermediate layer; the first intermediate layer and the second intermediate layer partially overlap to form a first area, a second area, a third area, and a fourth area.

In an embodiment, a plurality of intermediate layers are arranged, and the intermediate layers include a first intermediate layer and a second intermediate layer; the first intermediate layer and the second intermediate layer are separate from each other.

In an embodiment, a plurality of intermediate layers are arranged, and the intermediate layers include a first intermediate layer and/or a second intermediate layer; the first intermediate layer extends axially and the second intermediate layer extends circumferentially.

Compared with the existing art, according to the above-mentioned balloon sheath, the inflation chamber is arranged inside the main body, thereby reducing the outer diameter of the balloon sheath. In addition, when the inflation pressure of the balloon gradually increases and is less than a preset value, the balloon expands to occlude blood flow. At this point, the balloon gradually expands to fill a gap between the main body and a blood vessel, while still allowing an instrument to pass through the main body normally. When the inflation pressure reaches the preset value, it means that the internal pressure of the balloon reaches a strength capable of deforming a distal end section of the main body. At this point, the balloon not only fills the gap between the main body and the blood vessel but also forces a corresponding position at the distal side of the main body to squeeze and deform towards the inner side, such that the inner wall of the main body is tightly attached to the instrument, thereby achieving the effect of simultaneously fixing the instrument and occluding the blood flow. Furthermore, when this solution is applied to blood flow diversion, it can also accomplish the following: when the balloon first expands to fill the gap between the main body and the blood vessel, the main body is communicated with the blood vessel to participate in blood flow diversion (namely, cooperating to achieve reverse blood flow); when the balloon continues to inflate and the internal pressure gradually increases to deform the main body, the diameter of the diversion passage gradually decreases, so as to reduce the flow rate of the blood flow, until the blood flow finally stops.

### Brief Description of the Drawings

FIG. 1 is a schematic structural diagram of a balloon sheath according to an embodiment of the present invention.
FIG. 2 is a schematic diagram of a first operation of a balloon sheath according to an embodiment of the present invention.
FIG. 3 is a schematic diagram of the operation of a balloon sheath according to an embodiment of the present invention.
FIG. 4 is a schematic structural diagram of a balloon sheath according to another embodiment of the present invention.
FIG. 5 is a schematic diagram of a cross-section taken from a left side to a right side along line A-A in FIG. 4.
FIG. 6 is a schematic structural diagram of a first cross-section of a balloon sheath according to yet another embodiment of the present invention.
FIG. 7 is a schematic diagram of a cross-section taken from a left side to a right side along line B-B in FIG. 6.
FIG. 8 is a schematic structural diagram of a second cross-section of a balloon sheath according to yet another embodiment of the present invention.
FIG. 9 is a schematic structural diagram of a balloon sheath according to still yet another embodiment of the present invention.
FIG. 10 is a schematic diagram of a cross-section taken from a left side to a right side along line C-C in FIG. 9.

### Detailed Description of the Invention

To make the object, technical solutions, and advantages of the present invention more apparent, the present invention will be described in detail with reference to accompanying drawings and embodiments. It should be understood that specific embodiments described herein are merely illustrative of the present invention and are not intended to limit the present invention.

It should be noted that in the field of interventional medical instruments, generally, an end of a medical instrument implanted in a human or animal body which is closer to the operator is called "a proximal end", and an end which is farther from the operator is called "a distal end"; the "proximal end" and "distal end" of any component of the medical instrument are defined according to this principle. "Axial direction" generally refers to the length direction of a medical instrument as it is transported, and "radial direction" generally refers to the direction of a medical instrument perpendicular to its "axial direction"; the "axial direction" and the "radial direction" of any component of the medical instrument are defined according to this principle. "Connection" in the embodiments includes the case where two components are directly connected and indirectly connected through other components.

The technical solutions of the present invention will be described in further detail below with reference to specific embodiments.

### Embodiment 1

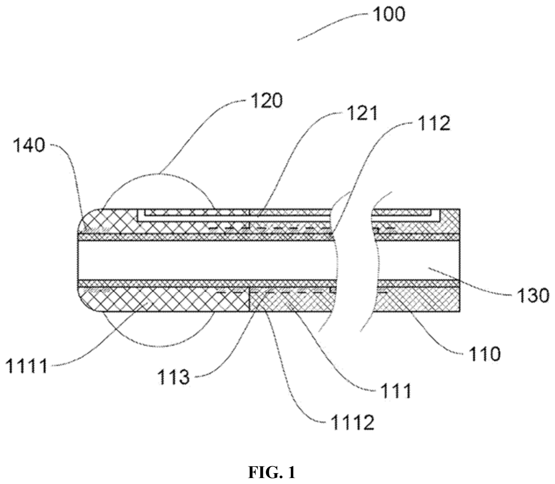

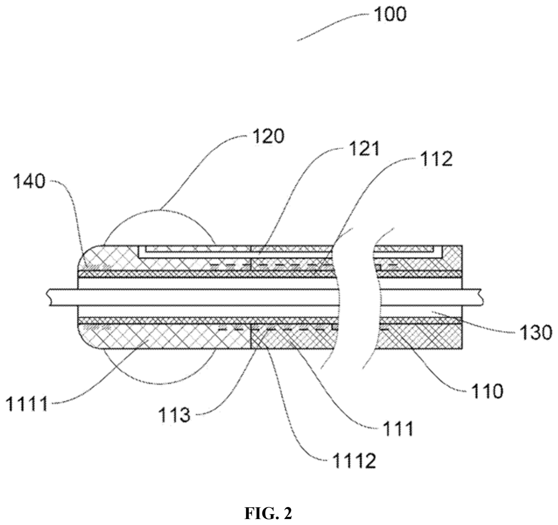

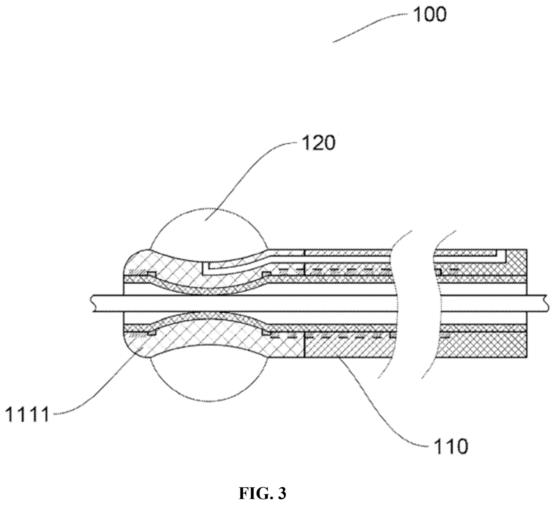

With reference to FIG. 1 to FIG. 3, FIG. 1 is a schematic structural diagram of a balloon sheath 100 according to embodiment 1 of the present invention; FIG. 2 is a schematic diagram of a first operation of a balloon sheath 100 according to embodiment 1 of the present invention; FIG. 3 is a schematic diagram of a second operation of a balloon sheath 100 according to embodiment 1 of the present invention. The balloon sheath 100 is provided with a guide wire in the first operation state. From the first operation state to the second operation state, a balloon in the balloon sheath 100 is inflated.

In this embodiment, the balloon sheath 100 includes a tubular main body 110, and the main body 110 includes an outer layer 111 and an inner layer 112. The balloon sheath 100 further includes a balloon 120 located mostly outside the outer layer 111 and an inner cavity 130 on an inner side of the inner layer 112 for transporting a medical instrument. The main body 110 mainly serves as a passage for transporting the instrument. The outer layer 111 of the main body 110 needs to have good biocompatibility and a certain strength due to the need for intervention into the human body. Therefore, in this embodiment, the outer layer 111 preferably includes a PEBAX material. The middle passage of the inner layer 112 is the transportation passage of the instrument, and therefore, in order to smooth the transportation process, the inner layer 112 preferably includes a PTFE film in this embodiment. In order to increase the strength of the inner layer 112, avoid the occurrence of creases in the inner layer 112 to affect the transportation of the instrument, and avoid influencing the flexibility of the main body 110 due to an excessive increase in the strength, a spring tube 113 is wound around an outer surface of the inner layer 112, and the outer layer 111 surrounds outer sides of the inner layer 112 and the spring tube 113.

In this embodiment, a balloon 120 is provided at a distal end of the main body 110, and the balloon 120 is a semi-compliant balloon. When the inflation pressure increases, the diameter of the balloon 120 also increases, so that the balloon 120 abuts against an inner wall of a blood vessel to fully occlude the blood vessel and stop the blood flow. When the balloon 120 is inflated, the main body 110 is located at the center of the balloon 120, so that the main body 110 does not touch the inner wall of the blood vessel when the balloon 120 occludes the blood vessel, thereby protecting the blood vessel from damage.

In this embodiment, as the balloon 120 gradually inflates, it occludes the blood flow while still allowing the instrument to pass through normally; when the internal inflation pressure of the balloon 120 is greater than a prescribed value, the balloon 120 circumferentially compresses a portion of the main body 110, causing the main body 110 to deform and be tightly attached to the instrument, thereby achieving the effect of fixing the instrument and hemostasis.

When the inflation pressure gradually increases, the balloon expands to occlude the blood flow, while still allowing the instrument to pass through normally; when the inflation pressure exceeds the prescribed value, the balloon longitudinally compresses a portion of the sheath, so that the longitudinal inner wall (without longitudinal spring support) of the sheath is tightly attached to and fixes the instrument, and the lateral inner wall (with lateral spring support) of the sheath forms a certain space with the instrument, so as to allow reverse blood flow transport, thereby achieving the purposes of fixing the instrument and facilitating blood flow diversion.

Notably, in this embodiment, the inflation chamber 121 of the balloon 120 penetrates the interior of the outer layer 111 to have a significantly smaller volume relative to other designs that additionally cover the inflation chamber of the balloon to the exterior of the sheath, and precisely, this embodiment allows the main body 110 to have a smaller outer diameter and a larger outer diameter to allow for a larger instrument transportation space while maintaining a good inflation rate.

In this embodiment, a marking ring 140 is provided at the distal end of the main body 110 to facilitate positioning of the balloon sheath 100 after implantation.

In addition, in this embodiment, the proximal end and the distal end of the main body 110 have different strengths. Specifically, the distal end of the main body 110 has better flexibility to prevent the end from damaging the blood vessel during the transportation of the instrument and the introduction into the human body, and the proximal end of the main body 110 has a stronger supporting force to maintain the overall strength of the main body 110 and prevent the force transmitted from the proximal end from being not transmitted to the distal end when the main body 110 needs to be moved to a predetermined position. Preferably, the outer layer 111 of the main body 110 includes a first section 1111 and a second section 1112, where the first section 1111 is selected from the material type of PEBAX35D and the second section 1112 is selected from the material type of PEBAX63D.

For this embodiment, the balloon 120 is filled to expand and to occlude the blood vessel by injecting a medium into the inflation chamber 121 of the balloon 120, while simultaneously allowing instrument transportation and blood flow diversion through the inner cavity 130.

With reference to FIG. 2, when the inflation pressure of the balloon 120 gradually increases and is less than a preset value, the balloon 120 expands to occlude the blood flow. At this point, the balloon 120 gradually expands to fill a gap between the main body 110 and the blood vessel, while still allowing an instrument to pass through the main body normally. It should be noted that the preset value is determined in combination with the pressure strength of the balloon and the overall strength of the position of the distal end (namely, the first section 1111) of the main body 110.

When the inflation pressure reaches the preset value, it means that the internal pressure of the balloon 120 reaches a strength capable of deforming the distal end section of the main body 110, as shown in FIG. 3. At this point, the balloon 120 not only fills the gap between the main body 110 and the blood vessel but also forces the corresponding position at the distal side of the main body 110 to squeeze and deform towards the inner side, such that the inner wall of the main body 110 is tightly attached to the instrument, thereby achieving the effect of simultaneously fixing the instrument and occluding the blood flow.

Since it is possible to control the expanding volume of the balloon 120, when this embodiment is applied to blood flow diversion, namely, the inner cavity 130 serves as a passage for blood flow diversion. It can also accomplish the following: when the balloon 120 first expands to fill the gap between the main body 110 and the blood vessel, the inner cavity 130 is communicated with the blood vessel to participate in blood flow diversion (namely, cooperating to achieve reverse blood flow); when the balloon 120 continues to inflate and the internal pressure gradually increases to deform the main body 110, the diameter of the passage of the inner cavity 130 gradually decreases, so as to reduce the flow rate of the blood flow, until the blood flow finally stops, thereby achieving the effect of controlling the blood flow diversion rate, and eliminating the need for an intermediate piece to be introduced for controlling the flow rate.

In this embodiment, in order to avoid the excessive strength of the main body 110 at the location corresponding to the balloon 120, this embodiment preferably does not arrange the spring tube 113 in the area of the inner layer 112 corresponding to the balloon 120, namely, the strength of the main body 110 in the area of the balloon 120 is less than the strength of the main body 110 in the proximal side area.

In another embodiment, an auxiliary passage is arranged between the balloon 120 and the inner layer 112, namely, the outer surface of the inner layer 112 is communicated with the balloon 120. When the balloon 120 is inflated, the pressure of the balloon 120 can also directly act on the inner layer 112. This avoids deformation of the outer layer 111 causing deformation in other areas and also reduces the difficulty of deforming the main body 110.

### Embodiment 2

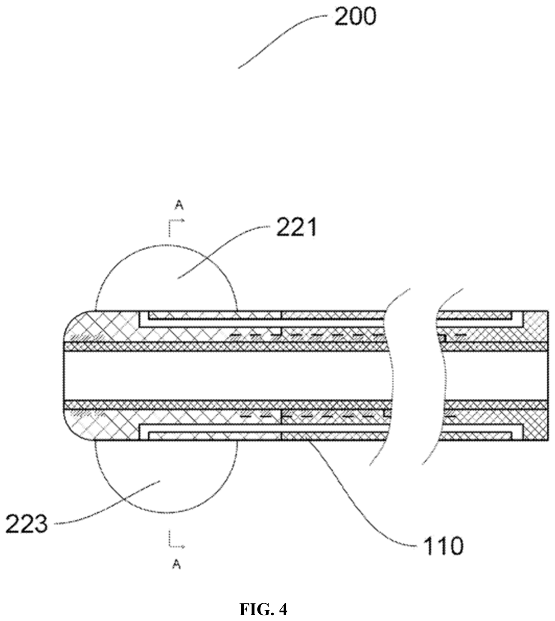

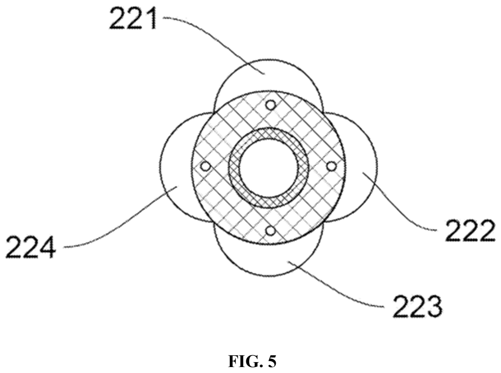

The only difference between this embodiment and embodiment 1 is that in this embodiment, a plurality of balloons are arranged in a circumferential direction at a distal end of a main body 110. With reference to FIG. 4 and FIG. 5, FIG. 4 is a schematic structural diagram of a balloon sheath 200 in embodiment 2 of the present invention, and FIG. 5 is a schematic diagram of a cross-section taken from a left side to a right side along line A-A in FIG. 4. The plurality of balloons are arranged in a circumferential direction at the distal end of the main body 110 of the balloon sheath 200 and include a balloon 221, a balloon 222, a balloon 223, and a balloon 224. Each balloon has a separate inflation chamber, so that the balloon 221, the balloon 222, the balloon 223, and the balloon 224 can be individually controlled.

By inflating the different balloons, the fixation of the instrument within the balloon sheath 200 and the flow of reverse blood flow can be flexibly controlled, with better adaptability than the single balloon approach in embodiment 1.

### Embodiment 3

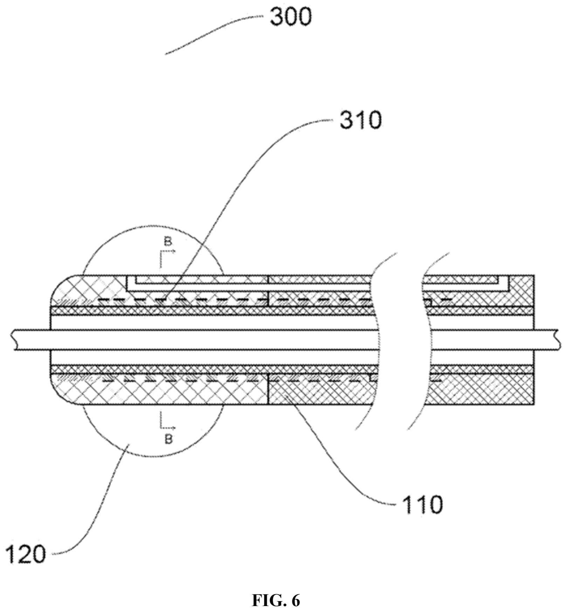

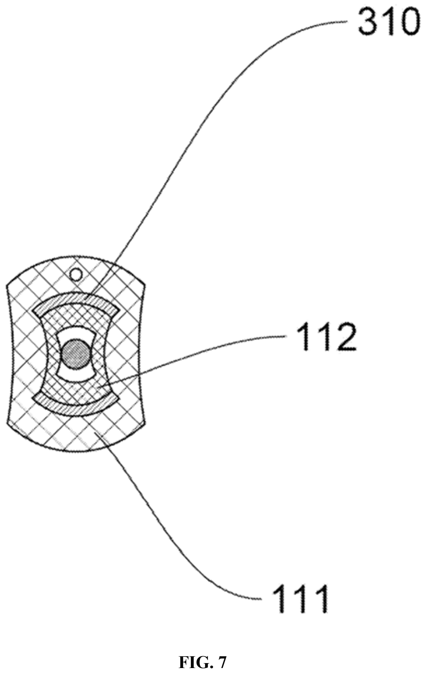

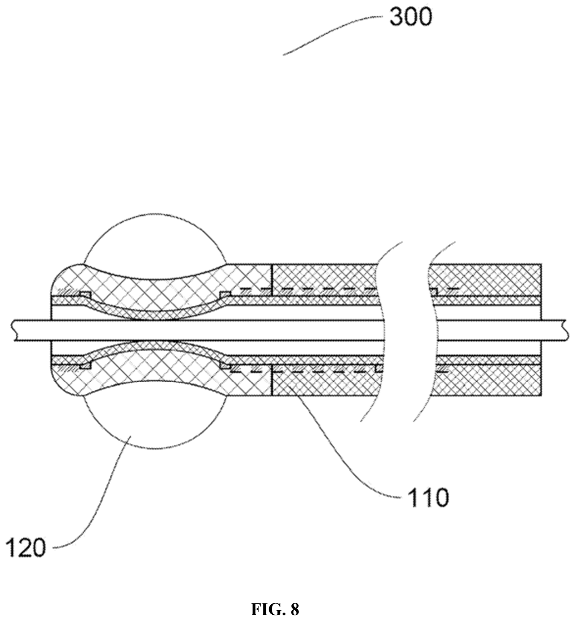

The only difference between this embodiment and embodiment 1 is that in this embodiment, an intermediate layer is arranged at the distal end of the main body 110. With reference to FIG. 6 to FIG. 8, FIG. 6 is a schematic structural diagram of a first cross-section of a balloon sheath 300 in embodiment 3 of the present invention, FIG. 7 is a schematic diagram of a cross-section from a left side to a right side taken along line B-B in FIG. 6, and FIG. 8 is a schematic structural diagram of a second cross-section of the balloon sheath 300 in embodiment 3 of the present invention, where the first cross-section and the second cross-section are longitudinal cross-sections, and the first cross-section and the second cross-section are perpendicular; FIG. 6 and FIG. 7 are both in a state where the balloon 120 is inflated, and the purpose of this is to reflect the cover area and function of the intermediate layer 310.

The intermediate layer 310 partially covers the surface of the inner layer 112 in a circumferential direction to enhance the strength of the inner layer 112 in certain areas. In this embodiment, a spring tube can be used, with the specific distribution location referenced in FIG. 7.

It should be noted that due to the presence of the intermediate layer 310, after the balloon 120 is subjected to a higher inflation pressure, the area of the main body 110 that deforms inward is the area that is not covered by the intermediate layer 310, because the intermediate layer 310 has a supporting force that is capable of counteracting the pressure of the balloon 120. The purpose of this is to allow a certain space between the inner wall of the balloon sheath 300 (the area supported by the intermediate layer) and the instrument to allow reverse blood flow transport, but other areas can still deform normally, thereby achieving the purposes of fixing the instrument and facilitating blood flow diversion.

In this embodiment, the intermediate layer 310 can be selected from an elastic piece such as a flexible spring sheet or mesh sheet, and its purpose can also be that, instead of leaving a certain space between the inner wall of the balloon sheath 300 (the area supported by the intermediate layer 310) and the instrument at all times, the area arranged with the intermediate layer 310 needs a relatively higher pressure to achieve deformation with respect to the area not arranged with the intermediate layer 310, so as to achieve the phasing of deformation. For example: a medium is injected into the balloon 120 to gradually increase the pressure to a first stage, and the balloon completes the occlusion of the gap between the outside of the main body 110 and the blood vessel; when the pressure of the balloon 120 is increased to a second stage, the area of the main body 110 without the intermediate layer 310 is deformed, so that a certain space is left between the inner wall of the balloon sheath 300 (the area supported by the intermediate layer) and the instrument, allowing reverse blood flow transport, but the other areas can still deform normally, thereby achieving the purposes of fixing the instrument and facilitating blood flow diversion; when the pressure of the balloon 120 is increased to a third stage, the area of the intermediate layer 310 also starts to deform, and the gap between the inner wall of the main body 110 and the instrument is filled, thereby fixing the instrument and stopping the blood flow transport.

It should be noted that, in the case where the balloon 120 acts directly on the inner layer 112 (if the outer layer 111 is not deformed), the intermediate layer 310 needs to be arranged correspondingly on the inner layer 112.

### Embodiment 4

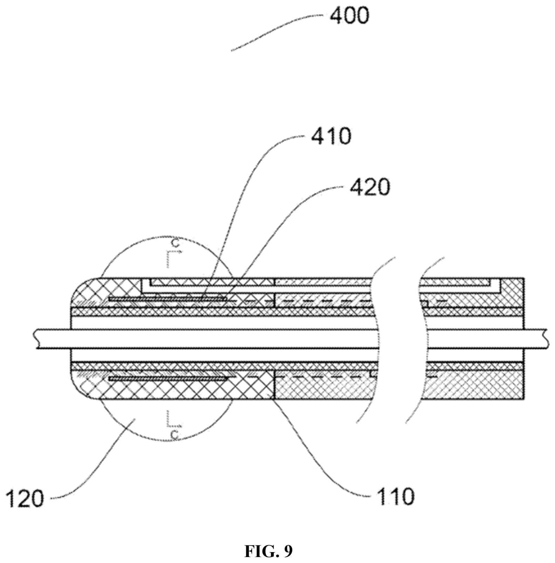

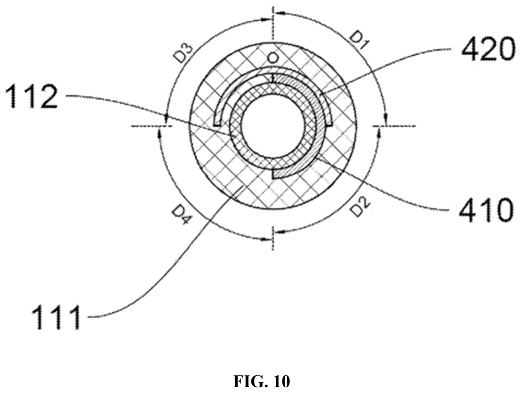

The only difference between this embodiment and embodiment 3 is that in this embodiment, a plurality of intermediate layers are arranged. With specific reference to FIG. 9 and FIG. 10, FIG. 9 is a schematic structural diagram of a balloon sheath 400 in embodiment 4 of the present invention, and FIG. 10 is a schematic diagram of a cross-section taken from a left side to a right side along line C-C in FIG. 9. In this embodiment, at least a first intermediate layer 410 and a second intermediate layer 420 are arranged in the main body 110, where the first intermediate layer 410 and the second intermediate layer 420 partially overlap to cover the main body 110 in a circumferential direction.

In this embodiment, the first intermediate layer 410 adopts a spring tube structure, and the second intermediate layer 420 adopts a spring sheet structure. In this embodiment, the strength of the first intermediate layer 410 is limited to be greater than the strength of the second intermediate layer 420. When the first intermediate layer 410 and the second intermediate layer 420 are distributed in the manner shown in FIG. 9 and FIG. 10, at least the following four areas are formed:
a first area D1, where the first area D1 includes both the first intermediate layer 410 and the second intermediate layer 420, and the required deformation pressure for the first area D1 is the highest;
a second area D2, where the second area D2 only includes the first intermediate layer 410, and the required deformation pressure for the second area D2 is less than that for the first area D1;
a third area D3, where the third area D3 only includes the second intermediate layer 420, and the required deformation pressure for the third area D3 is less than that for the second area D2; and
a fourth area D4, where the fourth area D4 is not covered by the intermediate layers, and the required deformation pressure for the fourth area D4 is the lowest.

With this design, the inflation stages of the balloon 120 can be divided into four stages as follows:
in the first stage, a medium is injected into the balloon 120 to gradually increase the pressure, the balloon completes the occlusion of the gap between the outside of the main body 110 and the blood vessel, and at this time, blood is allowed to flow in reverse through the interior of the main body 110;
in the second stage, when the pressure of the balloon 120 is continuously increased, the fourth area D4 in the main body 110 is deformed, and the main body 110 in the fourth area D4 moves towards the inner side; the inner wall of the main body 110 (areas supported by the intermediate layers) in the other areas still has a certain space from the instrument; the space for reverse blood flow transport decreases, which results in a reduced flow rate of reverse blood flow, thereby controlling the blood flow diversion rate to the value in the second stage; if the fourth area D4 has parts oppositely distributed, the function of fixing the instrument (by abutting against the instrument from opposite positions) is also provided.
in the third stage, the pressure of the balloon 120 is continuously increased on the basis of the second stage; the third area D3 also begins to deform, and the space for the reverse blood flow transport further decreases, which results in a further reduction in the flow rate of the reverse blood flow, thereby controlling the blood flow diversion rate to the value of the third stage;
in the fourth stage, the pressure of the balloon 120 is continuously increased on the basis of the third stage; the second area D2 also begins to deform, and the space for the reverse blood flow transport further decreases, which results in a further reduction in the flow rate of the reverse blood flow, thereby controlling the blood flow diversion rate to the value of the fourth stage; and
in the fifth stage, the pressure of the balloon 120 is continuously increased on the basis of the fourth stage; the first area D1 also begins to deform, and the space for the reverse blood flow transport further decreases, which results in a further reduction in the flow rate of the reverse blood flow, until the main body 110 of the first area D1 also abuts against the instrument, so that the inner wall of the main body 110 completely abuts against the instrument, thereby completely fixing the instrument, and at this time, the blood flow diversion rate is almost zero, and the blood flow diversion stops.

Therefore, a more complex multi-stage regulation of the reverse blood flow transport is achieved by the two intermediate layers partially overlapping each other, as in this embodiment.

In another embodiment, the structural arrangement of the first intermediate layer 410 and the second intermediate layer 420 is the same, but their thicknesses are different, which can also achieve technical effects similar to those in embodiment 4. However, this may increase the overall thickness of the sheath or cause the center of gravity of the distal end of the main body 110 to deviate from the axis.

In another embodiment, the areas covered by the first intermediate layer 410 and the second intermediate layer 420 do not overlap at all, namely, they are separated from each other.

In another embodiment, the main body 110 includes a plurality of first intermediate layers 410 and/or a plurality of second intermediate layers 420.

In another embodiment, the main body 110 includes a plurality of first intermediate layers 410 and/or a plurality of second intermediate layers 420. The first intermediate layers 410 extend axially and the second intermediate layers 420 extend circumferentially. This arrangement achieves multiple areas with varying strengths that are uniformly distributed and can deform along with the balloon 120.

It should be noted that the various embodiments of the present invention, as well as the technical features of each embodiment, can be combined in any manner. To keep the description concise, not all possible combinations of the technical features in the above embodiments have been described. However, as long as the combinations of these technical features do not contradict each other, they should be considered to be within the scope of the specification.

The embodiments described above merely represent several implementations of the present invention. The description is specific and detailed, but it should not be construed as limiting the scope of the present invention. It should be noted that for those skilled in the art, several variations and modifications can still be made without departing from the concept of the present invention, and these variations and modifications all fall within the scope of protection of the present invention. Therefore, the scope of protection of the present invention shall be subject to the appended claims.

## Claims

1. A balloon sheath, comprising a tubular main body and a balloon located at a distal end of the main body, wherein the main body internally comprises an inflation chamber; the inflation chamber is communicated with the balloon, and an inner wall of the main body can be driven to move towards an inner side when the balloon expands.

2. The balloon sheath according to claim 1, wherein the main body comprises an inner layer and an outer layer, and a spring tube is arranged between the inner layer and the outer layer.

3. The balloon sheath according to claim 2, wherein the spring tube avoids an area of the main body where the balloon is located.

4. The balloon sheath according to claim 2, wherein the balloon is communicated with an outer surface of the inner layer.

5. The balloon sheath according to claim 1, wherein a plurality of balloons are arranged, and the plurality of balloons are distributed in a circumferential direction at the distal end of the main body.

6. The balloon sheath according to claim 1, wherein the main body comprises at least one intermediate layer, and the intermediate layer at least partially covers an area where the balloon is located in a circumferential direction.

7. The balloon sheath according to claim 6, wherein the intermediate layer comprises one or more of a spring tube, a spring sheet, or a mesh sheet.

8. The balloon sheath according to claim 6, wherein a plurality of intermediate layers are arranged, and the intermediate layers comprise a first intermediate layer and a second intermediate layer; the first intermediate layer and the second intermediate layer partially overlap to circumferentially form a first area, a second area, a third area, and a fourth area.

9. The balloon sheath according to claim 6, wherein a plurality of intermediate layers are arranged, and the intermediate layers comprise a first intermediate layer and a second intermediate layer; the first intermediate layer and the second intermediate layer are separate from each other.

10. The balloon sheath according to claim 6, wherein a plurality of intermediate layers are arranged, and the intermediate layers comprise a first intermediate layer and/or a second intermediate layer; the first intermediate layer extends axially and the second intermediate layer extends circumferentially.
